# EUROPEAN PATENT APPLICATION

(11) **EP 1 795 517 A1**
(43) Date of publication of application: **13.06.2007**
(21) Application number: 05775119.0
(22) Date of filing: 29.08.2005
(51) Int. Cl.: C07C 7/08, C07C 11/167

(54) **APPARATUS FOR SEPARATION AND PURIFICATION OF UNSATURATED HYDROCARBON AND METHOD OF SEPARATION AND PURIFICATION**

(30) Priority: 31.08.2004 JP 2004252581
(71) Applicant: ZEON CORPORATION, Tokyo 100-8246 (JP)
(72) Inventor: KANAUCHI, Masanobu, Chiyoda-ku, Tokyo 100-8246 (JP); ARIMORI, Yasuhiko, Chiyoda-ku, Tokyo 100-8246 (JP)
(74) Representative: Vossius & Partner
(86) International application number: PCT/JP2005/015665
(87) International publication number: WO 2006/025330

(57) **Abstract**

For example, a separation and purification apparatus having an extractive distillation tower 4 for separating and purifying butadiene, impurity concentration sensors 32, 34 for detecting the concentrations of specific impurities other than butadiene, a target material concentration sensor for detecting the concentration of butadiene in the extractive distillation tower, and a differential pressure sensor 30 for detecting the differential pressure between the top and bottom of the extractive distillation tower 4 and a separation and purification method. The method calculates a concentration of a specific impurity after a predetermined time, a concentration of butadiene at the top, and a forecasted value of the differential pressure between the top and bottom based on the sensors and controls operations based on the forecasted values by a concentration predictive control means 60. It controls a feedstock flow rate control valve 21a controlling the rate of feedstock fed to the extractive distillation tower 4, a load detecting means 61 for detecting the load of the extractive distillation tower, and a feedstock flow rate control valve 21a by a load control means 62 in accordance with detection values detected by the load detecting means 61.

## Description

### TECHNICAL FIELD

The present invention relates to a separation and purification apparatus and separation and purification method of unsaturated hydrocarbons.

### BACKGROUND ART

1,3-butadiene, isoprene, and other conjugated dienes are generally separated and purified as unsaturated hydrocarbons by extractive distillation using a solvent from a C₄ fraction or C₅ fraction obtained by cracking naphtha and separating the ethylene, propylene, and other C₂ and C₃ hydrocarbons (see Patent Documents 1 to 4)

Normally, this extractive distillation is performed using an apparatus comprised of an extractive distillation tower and stripping tower. Conjugated dienes, which dissolve relatively easily in the solvents, in the C₄ fraction or C₅ fraction, are taken out as mixtures with the solvents from the bottom of the extractive distillation tower and sent to the stripping tower, where the conjugated dienes and solvents are separated. The solvents are then returned to the extractive distillation tower.

In the conventional separation and purification apparatus and separation and purification method for conjugated dienes, the general practice has been to control the feed rate of the solvent to the extractive distillation tower, control the flow rate of part of the residual components of the feedstock taken out from the top of the extractive distillation tower (residuum of feedstock after conjugated dienes have been extracted) and reflux it to the extractive distillation tower, control the bottom temperature of the extractive distillation tower, etc. to separate and purify a stable quality of conjugated dienes.

With such a conventional apparatus and method, however, when the composition of the feedstock fed to the extractive distillation tower varied, the concentration of the target conjugated dienes taken out from the tower varied. Consequently, it was difficult to take out a stable quality of conjugated dienes.

Note that to take out an extract of a high concentration and constant concentration of conjugated dienes from the extractive distillation tower, it is preferable to return the extract taken out from the bottom of the extractive distillation tower to the extractive distillation tower and control the return ratio. If the return ratio to the extractive distillation tower, however, is not allowed to fluctuate in accordance with the ratio of the solvent, the bottom temperature, the bottom pressure, the ratio of the feedstock fed, the concentration of the conjugated dienes in the feedstock, etc., it is not possible to maintain a constant concentration of the target butadiene, isoprene, or other conjugated dienes and concentration of other specific impurities in the extractive distillation tower. Further, it is close to impossible for an operator to manually handle this control procedure. Therefore, at the present time, priority is given to ease of operation. The return ratio is not controlled, but the flow rate to the next process is controlled and the surplus is returned. Therefore, there was a large fluctuation in the conjugated dienes taken out from the extractive distillation tower. In particular, if there is a large fluctuation in concentration of the conjugated dienes taken out in the first extractive distillation tower used for the separation and purification apparatus of the conjugated dienes, increasing the purity of the conjugated dienes in the subsequent processes becomes difficult and stably obtaining high purity conjugated dienes becomes difficult.

Therefore, Patent Document 5 proposes technology for detecting a change in impurity concentration close to the bottom of the extractive distillation tower and a change in the concentration of conjugated dienes in the gas discharged from the top of the extractive distillation tower and, in accordance with the changes, controlling the feed rate of the solvent to the extractive distillation tower, controlling the return flow rate from the stripping tower to the extractive distillation tower, controlling the reflux ratio at the top of the extractive distillation tower, and controlling the bottom temperature of the extractive distillation tower so as to extract a certain concentration of conjugated dienes. However, with this method, there was the problem that sufficient conjugated dienes production could not be obtained.
Patent Document 1: Japanese Patent Publication (B) No. 45-17405
Patent Document 2: Japanese Patent Publication (B) No. 45-17411
Patent Document 3: Japanese Patent Publication (B) No. 47-41323
Patent Document 4: Japanese Patent Publication (A) No. 56-83421
Patent Document 5: Japanese Patent Publication (A) No. 11-349499

### DISCLOSURE OF THE INVENTION

An object of the present invention is to provide a separation and purification apparatus and separation and purification method of unsaturated hydrocarbons having a high production capacity which enable a target conjugated diene or other unsaturated hydrocarbons to be stably taken out at a predetermined concentration regardless of variations in the components of the feedstock.

To achieve the above object, the inventors engaged in in-depth studies and as a result discovered that the capacity of the equipment used not being utilized to the maximum extent is the reason for the drop in production capacity of the target unsaturated hydrocarbon and completed the present invention based on this discovery.

That is, the first separation and purification apparatus for an unsaturated hydrocarbon of the present invention comprising:
an extractive distillation tower fed with feedstock containing unsaturated hydrocarbons and a solvent and distilling the feedstock and solvent to separate and purify a target unsaturated hydrocarbon which is part of the unsaturated hydrocarbons;
an impurity concentration detecting means for detecting a concentration of a specific impurity other than the target unsaturated hydrocarbon at the extractive distillation tower or another tower connected to the extractive distillation tower;
a target material concentration detecting means for detecting a concentration of the target unsaturated hydrocarbon at the extractive distillation tower or another tower connected to the extractive distillation tower;
a means for taking out a fluid containing the target unsaturated hydrocarbon from the bottom of the extractive distillation tower, a means for returning part of the fluid taken out to the extractive distillation tower, and a return ratio control means for controlling a return ratio returned to the extractive distillation tower;
a solvent ratio control means for controlling a feed rate of the solvent;
a reflux ratio control means for taking out a residual component of the feedstock from a top of the extractive distillation tower and controlling a reflux ratio of the residual component refluxed to the extractive distillation tower;
a bottom temperature control means for controlling a bottom temperature of the extractive distillation tower;
a concentration predictive control means for calculating a forecasted value of the concentration of the specific impurity and a forecasted value of the concentration of the target unsaturated hydrocarbon after a predetermined time based on values detected by the impurity concentration detecting means and the target material concentration detecting means and controlling the return ratio control means and the reflux ratio control means based on the forecasted values;
a feedstock feed rate control means for controlling the rate of the feedstock fed to the extractive distillation tower;
a load detecting means for detecting a load of the extractive distillation tower; and
a load control means for controlling the feedstock feed rate control means in accordance with detection values detected by the load detecting means.

Further, the second separation and purification apparatus for an unsaturated hydrocarbon of the present invention comprising:
an extractive distillation tower fed with feedstock containing unsaturated hydrocarbons and a solvent and distilling the feedstock and solvent to separate and purify a target unsaturated hydrocarbon which is part of the unsaturated hydrocarbons;
an impurity concentration detecting means for detecting a concentration of a specific impurity other than the target unsaturated hydrocarbon at the extractive distillation tower or another tower connected to the extractive distillation tower;
a target material concentration detecting means for detecting a concentration of the target unsaturated hydrocarbon at the extractive distillation tower or another tower connected to the extractive distillation tower;
a means for taking out a fluid containing the target unsaturated hydrocarbon from the bottom of the extractive distillation tower, a means for returning part of the fluid taken out to the extractive distillation tower, and a return ratio control means for controlling a return ratio returned to the extractive distillation tower;
a solvent ratio control means for controlling a feed rate of the solvent;
a reflux ratio control means for taking out a residual component of the feedstock from a top of the extractive distillation tower and controlling a reflux ratio of the residual component refluxed to the extractive distillation tower;
a bottom temperature control means for controlling a bottom temperature of the extractive distillation tower;
a concentration predictive control means for calculating a forecasted value of the concentration of the specific impurity and a forecasted value of the concentration of the target unsaturated hydrocarbon after a predetermined time based on values detected by the impurity concentration detecting means and the target material concentration detecting means and controlling the return ratio control means, the solvent ratio control means, the reflux ratio control means, and the bottom temperature control means based on the forecasted values;
a feedstock feed rate control means for controlling the rate of the feedstock fed to the extractive distillation tower;
a load detecting means for detecting a load of the extractive distillation tower; and
a load control means for controlling the feedstock feed rate control means in accordance with a detection value detected by the load detecting means.

The effect of the present invention can be further increased by calculating the forecasted value of the concentration of the specific impurity after a predetermined time and the forecasted value of the concentration of the target unsaturated hydrocarbon and by controlling not only the return ratio control means and reflux ratio control means, but also the solvent ratio control means and the bottom temperature control means based on the forecasted values.

Further, a first method for separation and purification of an unsaturated hydrocarbon according to the present invention comprising the steps of:
distilling a feedstock containing a target unsaturated hydrocarbon and a solvent fed to an extractive distillation tower;
detecting a concentration of a specific impurity other than the target unsaturated hydrocarbon at the extractive distillation tower or another tower connected to the extractive distillation tower;
detecting a concentration of the target unsaturated hydrocarbon at the extractive distillation tower or another tower connected to the extractive distillation tower;
controlling a return ratio of part of a fluid containing the target unsaturated hydrocarbon taken out from a bottom of the extractive distillation tower and returned to the extractive distillation tower;
controlling a solvent ratio of the solvent fed to the extractive distillation tower;
controlling a reflux ratio of part of a residual component of the feedstock taken out from a top of the extractive distillation tower and refluxed to the extractive distillation tower;
controlling a bottom temperature of the extractive distillation tower;
calculating a forecasted value of the concentration of the specific impurity and a forecasted value of the concentration of the target unsaturated hydrocarbon after a predetermined time based on values detected by the impurity concentration detecting step and the target material concentration detecting step and controlling the return ratio and the reflux ratio based on the forecasted values;
controlling the feedstock feed rate of the feedstock fed to the extractive distillation tower;
detecting a load of the extractive distillation tower; and
controlling the feedstock feed rate in accordance with a detection value detected by the load detection step.

Further, a second method for separation and purification of an unsaturated hydrocarbon according to the present invention comprises the steps of:
distilling a feedstock containing a target unsaturated hydrocarbon and a solvent fed to an extractive distillation tower;
detecting a concentration of a specific impurity other than the target unsaturated hydrocarbon at the extractive distillation tower or another tower connected to the extractive distillation tower;
detecting a concentration of the target unsaturated hydrocarbon at the extractive distillation tower or another tower connected to the extractive distillation tower;
controlling a return ratio of part of a fluid containing the target unsaturated hydrocarbon taken out from a bottom of the extractive distillation tower and returned to the extractive distillation tower;
controlling a solvent ratio of the solvent fed to the extractive distillation tower;
controlling a reflux ratio of part of a residual component of the feedstock taken out from a top of the extractive distillation tower and refluxed to the extractive distillation tower;
controlling a bottom temperature of the extractive distillation tower;
calculating a forecasted value of the concentration of the specific impurity and a forecasted value of the concentration of the target unsaturated hydrocarbon after a predetermined time based on values detected by the impurity concentration detecting step and the target material concentration detecting step and controlling the return ratio, the solvent ratio, the reflux ratio, and the bottom temperature based on the forecasted values;
controlling the feedstock feed rate of the feedstock fed to the extractive distillation tower;
detecting a load of the extractive distillation tower; and
controlling the feedstock feed rate in accordance with a detection value detected by the load detection step.

The effect of the present invention can be further increased by calculating the forecasted value of the concentration of the specific impurity and the forecasted value of the concentration of the target unsaturated hydrocarbon after a predetermined time and by controlling not only the return ratio and the reflux ratio, but also the solvent ratio and the bottom temperature based on the forecasted values.

The load of an extractive distillation tower fluctuates in relation to not only the capacity of the extractive distillation tower itself, but also the capacities of the condenser, pump, reboiler, compressor, etc. In the present invention, the differences between the load of the extractive distillation tower changing along with time and the capacities of the extractive distillation tower itself, the condenser, pump, reboiler, compressor, and other equipment are calculated. Due to that, it is possible to maximize the processing rate while drawing out the capacities of the equipment in real time to the maximum extent.

The load may be calculated, for example, based on the data of the reflux ratio in the reflux line, the data of the distillation rate of the residual gas, the data of the flow rate of steam to the reboiler, the data of the steam pressure to the reboiler, the data of the return ratio to the extractive distillation tower, the data of the flow rate of the stripped gas, the data of the feed rate of the solvent to the extractive distillation tower, the data of the feed rate of the feedstock to the extractive distillation tower, the data of the pressure measured by the top pressure sensor of the extractive distillation tower, and the data of the top-bottom differential pressure detected by the differential pressure sensor of the extractive distillation tower. For example, the loads of the condenser and pump are calculated from the data of the reflux ratio and data of the distillation rate of the residual gas, the load of the reboiler is calculated from the data of the flow rate of the steam to the reboiler or the data of the steam pressure to the reboiler, and the load of the compressor is calculated from the data of the return ratio to the extractive distillation tower and the data of the flow rate of the stripped gas. Further, the load of the extractive distillation tower itself is calculated from the data of the reflux ratio, the data of the distillation rate of the residual gas, the data of the flow rate of steam to the reboiler, the data of the return ratio to the extractive distillation tower, the data of the solvent ratio, the data of the feed rate of the feedstock, the data of the pressure measured by the top pressure sensor, and the data of the top-bottom differential pressure detected by the differential pressure sensor.

The solvent (extraction solvent) fed along with the feedstock to the extractive distillation tower in the present invention may be dimethylformamide, diethylformamide, dimethylacetamide, and other N-alkyl substituted lower fatty acid amides, furfural, N-methylpyrrolidone, formylmorpholine, β-methoxypropionitrile, and other solvents used for extractive distillation of conjugated dienes from hydrocarbon fractions for example. These solvents may be used alone or may be used in mixtures of two or more types. Further, to adjust the boiling point, suitable amounts of water, methanol, etc. may be mixed. Further, it is also possible to jointly use polymerization inhibitors to inhibit polymerization of the conjugated dienes and acetylenes, antioxidants, defoaming agents, etc. with the solvent. As the solvent, an N-alkylsubstituted lower fatty acid amide or other amide compound is preferable.

The solvent is preferably fed to the extractive distillation tower from a solvent feed stage provided at a position higher than the position of feed stage of the feedstock containing the unsaturated hydrocarbons in the extractive distillation tower (feedstock feed stage).

Further, the polymerization inhibitor may be continuously fed from a position higher than the solvent stage. As the position higher than the solvent feed stage, for example, the side of the extractive distillation tower higher than the solvent feed stage or the inlet or outlet of the condenser at the top of the extractive distillation tower may be mentioned. Among these, installation at the inlet of the top condenser is preferable in that it enables the production of polymers inside the condenser to be suppressed and enables the production of polymers even in processes after the separator to be suppressed. The polymerization inhibitor is preferably one which stops or suppresses polymerization by a chain transfer reaction. The polymerization inhibitor is preferably a di-lower alkylhydroxylamine.

The feedstock used in the present invention is a petroleum fraction containing unsaturated hydrocarbons obtained by cracking naphtha, then separation. As the petroleum fraction, there are for example a C₂ fraction containing mainly C₂ hydrocarbons, a C₃ fraction containing mainly C₃ hydrocarbons, a C₄ fraction containing mainly C₄ hydrocarbons, and a C₅ fraction containing mainly C₅ hydrocarbons. Among these, a fraction increased in the concentration of the unsaturated hydrocarbons due to distillation etc. is preferred. Further, a fraction containing a large amount of conjugated dienes as unsaturated hydrocarbons is preferred. In particular, a C₄ fraction containing a large amount of butadiene and a C₅ fraction containing a large amount of isoprene is preferred.

Further, in the present invention, the "target unsaturated hydrocarbon" means an unsaturated hydrocarbon concentrated to 90 wt% or more, preferably 95 wt% or more, and taken out by the apparatus and method of the present invention among the unsaturated hydrocarbons contained in the petroleum fraction and is preferably butadiene or isoprene.

According to the apparatus and method of the present invention, it is possible to stably take out a target unsaturated hydrocarbon at a predetermined concentration regardless of variations in the feedstock components. Further, it is possible to calculate the differences between the load of equipment changing along with time and the capacity of equipment and possible to maximize the amount of processing (rate of production) in real time.

### BRIEF DESCRIPTION OF THE DRAWINGS

[FIG. 1] FIG. 1 is a schematic view of the overall configuration of a separation and purification apparatus for conjugated dienes;
[FIG. 2] FIG. 2 is a schematic view of a method of control of a first extractive distillation tower shown in FIG. 1;
[FIG. 3] FIG. 3 is a flow chart of the method of control of a load control means shown in FIG. 2;
[FIG. 4] FIG. 4 is a flow chart of the method of control of a concentration predictive control means shown in FIG. 2;
[FIG. 5] FIG. 5 is a graph of the relationship of the measurement data and control parameters

### BEST MODE FOR WORKING THE INVENTION

Below, the present invention will be explained based on the embodiments shown in the figures, but the present invention is not limited to these embodiments.

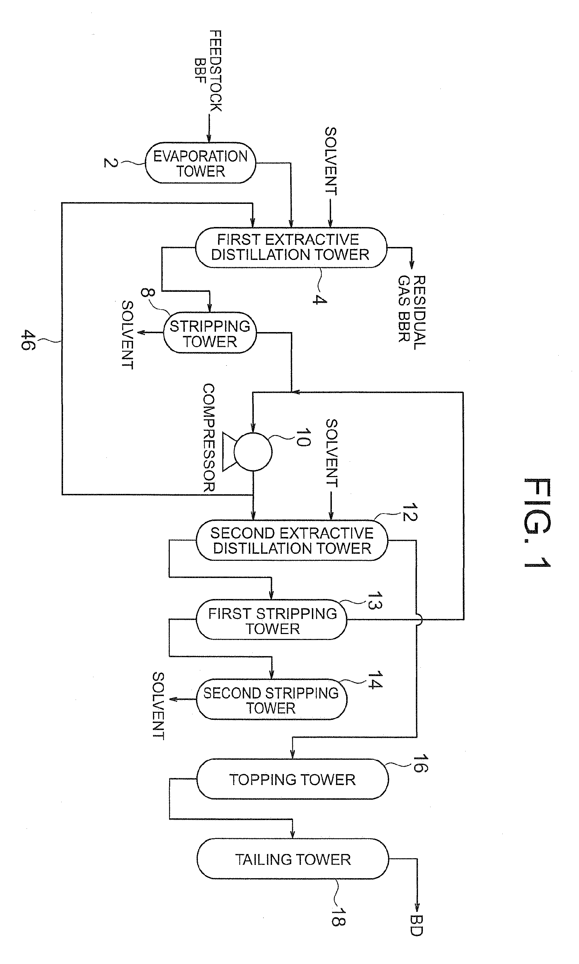

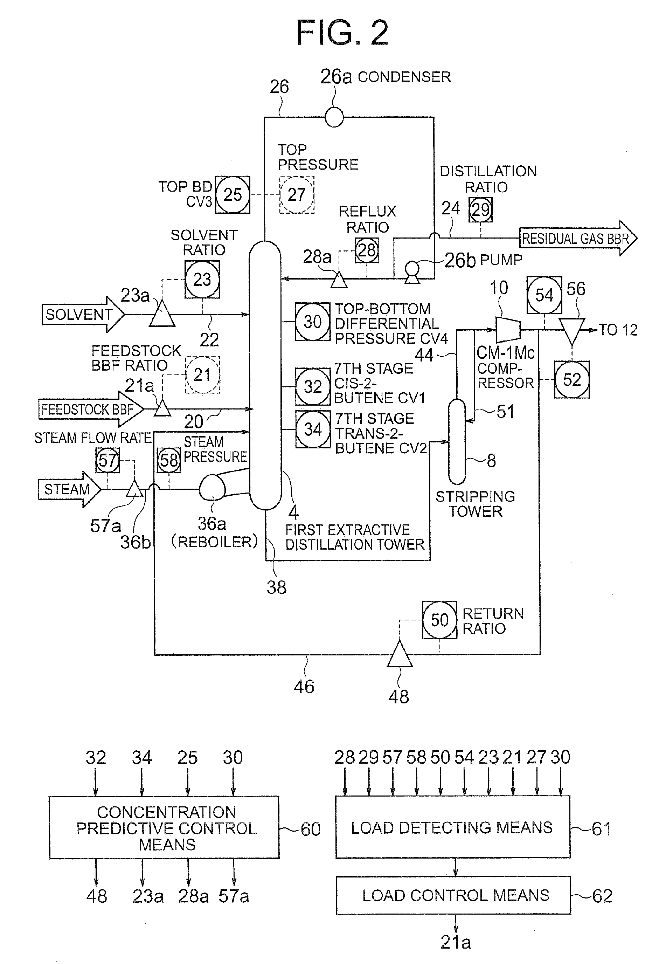

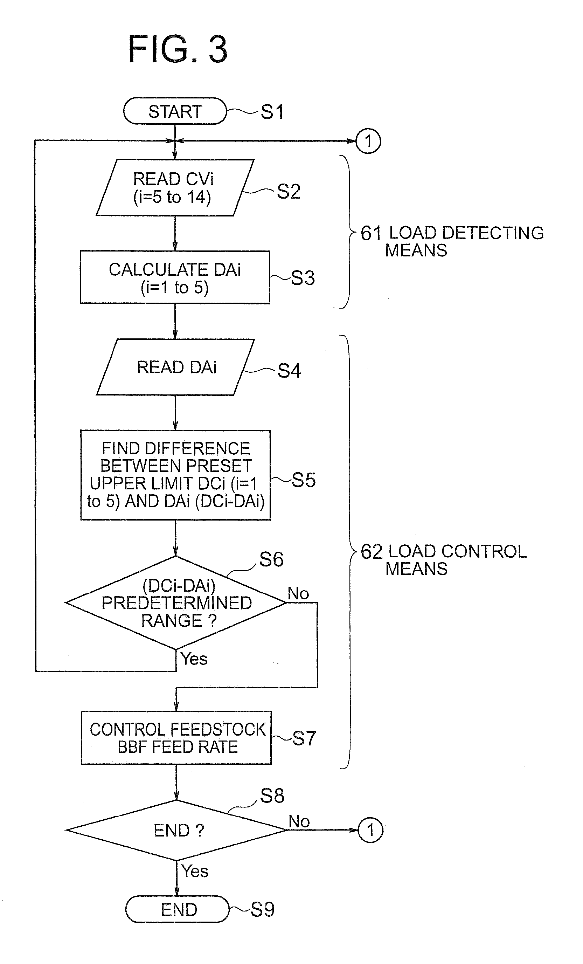

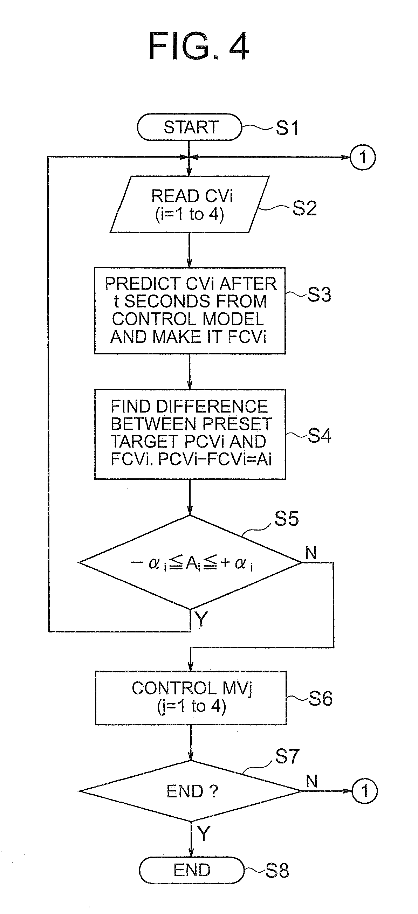

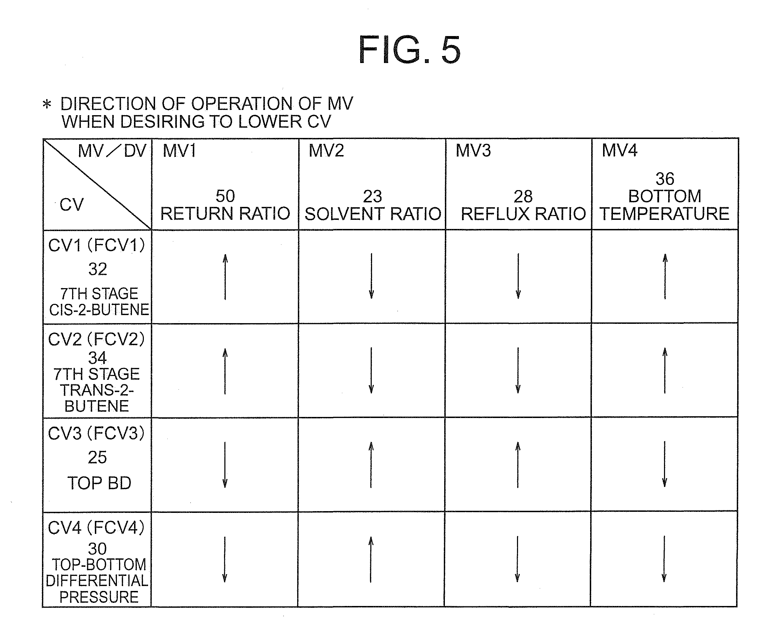

FIG. 1 is a schematic view of the overall configuration of a separation and purification apparatus for conjugated dienes; FIG. 2 is a schematic view of a method of control of a first extractive distillation tower shown in FIG. 1; FIG. 3 is a flow chart of the method of control of a load control means shown in FIG. 2; FIG. 4 is a flow chart of the method of control of a concentration predictive control means shown in FIG. 2; and FIG. 5 is a graph of the relationship of the measurement data and control parameters

In the present embodiment, the explanation will be given of the process of separation and purification of conjugated dienes from a C₄ fraction or C₅ fraction containing conjugated dienes as unsaturated hydrocarbons.

As shown in FIG. 1, the C₄ fraction or C₅ fraction (feedstock BBF) is first vaporized at an evaporation tower 2 and fed to a first extractive distillation tower 4. Further, the solvent is fed to a stage higher than the C₄ fraction or C₅ fraction feed position of the first extractive distillation tower 4. The solvent containing the conjugated dienes is fed from the bottom of the first extractive distillation tower 4 to a position several stages down from the top of a stripping tower 8. In the tower, the conjugated dienes and solvent are separated. The bottom temperature of the tower is normally controlled to become the boiling point of the solvent at a tower pressure of 0.5 to 5 atm. The conjugated dienes are taken out from the top of the stripping tower 8. Part is sent to a second extractive distillation tower 12 where it is purified, while the remainder is returned to the first extractive distillation tower 4. Solvent of normally 100 to 200°C is taken out from the bottom of the stripping tower 8.

In the present embodiment, by calculating the load of the first extractive distillation tower 4 (specifically, the loads of the condenser 26a, pump 26b, reboiler 36a, compressor 10, and first extractive distillation tower 4 itself shown in FIG. 2) and controlling the feed rate of the feedstock BBF to the first extractive distillation tower 4 in accordance with the calculated load, it is possible to increase to the maximum extent the load which fluctuates along with time and possible to operate the equipment at all times in the state of the maximum load. Along with this, in the present embodiment, by detecting the change in the concentration of the impurity near the bottom of the first extractive distillation tower 4 and the change of the concentration of the conjugated dienes in the gas discharged from the top of the first extractive distillation tower 4 and controlling the feed rate of the solvent fed to the first extractive distillation tower 4, controlling the return flow rate from the stripping tower 8 to the first extractive distillation tower 4, controlling the reflux ratio at the top of the first extractive distillation tower 4, and controlling the bottom temperature of the first extractive distillation tower 4 in accordance with these changes, it is possible to extract a constant concentration of conjugated dienes.

Below, a detailed explanation will be given of the process of separation and purification of butadiene from a C₄ fraction as an example.

As shown in FIG. 1, a C₄ component in naphtha (feedstock BBF) containing butadiene is fed to the evaporation tower 2 where the feedstock BBF is vaporized. In the evaporation tower 2, the feedstock BBF is vaporized by holding the tower temperature at preferably 20 to 80°C, more preferably 40 to 80°C, and holding the tower pressure at an absolute pressure of preferably 2 to 8 atm, more preferably 4 to 6 atm.

The feedstock BBF vaporized at the evaporation tower 2 is next fed to the first extractive distillation tower 4. The first extractive distillation tower 4 is fed with a solvent together with the vaporized feedstock BBF. The ratio of the solvent fed to the first extractive distillation tower 4 is controlled as explained later, but in general the solvent is fed to 100 to 1000 parts by weight, more preferably 200 to 800 parts by weight, with respect to 100 parts by weight of the feedstock BBF. The temperature of the solvent is preferably low since the solubility is high, but preferably is 10 to 100°C, more preferably 20 to 60°C since it affects the internal temperature of the first extractive distillation tower 4 or the change of the reflux ratio.

The solvent is not particularly limited so long as it enables dissolution and extraction of butadiene as one example of conjugated dienes, but specifically acetone, methylethylketone, dioxane, acetonitrile, methanol, ethanol, isopropanol, ethyleneglycol, propyleneglycol, N-ethylsuccinic acid imide, N-methylpyrrolidone, N-methyl-2-pyrrolidone, hydroxylethylpyrrolidone, N-methyl-5-methylpyrrolidone, furfural, 2-heptenone, dimethylformamide, dimethylacetamide, morpholine, N-formylmorpholine, N-methylmorpholin-3-one, sulforane, methylcarbitol, tetrahydrofuran, aniline, N-methyloxazolidone, N-methylimidazole, N,N'-dimethylimidazolin-2-one, 1-oxo-1-methylphosphorin, methylcyanoacetate, ethylacetoacetate, ethylacetate, dimethyl malonate, propylene carbonate, triethyl phosphate, diethylene glycol monomethyl ether, dimethyl sulfoxide, γ-butyrolactone, etc. may be mentioned. In the present embodiment, as the solvent, among these, amide compounds, in particular, dimethylformamide are preferable.

The solvent is fed to the first extractive distillation tower 4 from an extraction solvent feed stage provided at a position higher than the stage feeding the feedstock BBF (petroleum fraction feed stage) in the first extractive distillation tower 4.

At the top of the first extractive distillation tower 4 shown in FIGS. 1 and 2, the gas having a volatility of butadiene or more (solubility of butadiene or less) is separated, the residual gas of the feedstock BBF from which the butadiene component has been separated (hereinafter abbreviated as the "residual gas BBR") is taken out, and a high concentration butadiene extract is taken out from the bottom of the tower by controlling the bottom pressure of the first extractive distillation tower 4 to an absolute pressure of preferably 1 to 10 atm, more preferably 5 to 7 atm, and the bottom temperature to preferably 100 to 160°C, more preferably 110 to 130°C.

The amount of the C₄ fraction dissolved in the solvent taken out from the bottom of the first extractive distillation tower 4 is determined by the solvent ratio, temperature, and pressure at the bottom of the tower. Therefore, to take out a constant concentration butadiene extract from the bottom of the first extractive distillation tower 4, it is necessary to control the solvent ratio at the bottom of the first extractive distillation tower 4, the reflux ratio of the top, the bottom temperature, etc. Further, to increase the concentration of the butadiene extract taken out from the bottom of the first extractive distillation tower 4, as mentioned later, it is necessary to return the extract taken out from the bottom of the first extractive distillation tower 4 or, in accordance with need, part from which the solvent has been removed through the stripping tower 8, to the first extractive distillation tower 4. In the present embodiment, as explained later, the return ratio of the extract taken out from the bottom of the first extractive distillation tower 4 to the first extractive distillation tower 4 is also controlled.

The residual gas BBR taken out from the top of the first extractive distillation tower 4 is sent to a not shown residual component tank. Part of the residual gas BBR is condensed at a condenser 26a and refluxed by returning it to the top of the first extractive distillation tower 4. The reflux ratio of the residual gas BBR is also controlled as explained later.

At the bottom of the first extractive distillation tower 4 shown in FIGS. 1 and 2, an extract containing a high concentration of the target butadiene is taken out and sent to the stripping tower 8. In the stripping tower 8, the bottom pressure is held at an absolute pressure of 1 to 3 atm and the bottom temperature is held at 150 to 200°C. The solvent is separated from the extract and discharged from the bottom of the tower. At the top of the stripping tower 8, a stripped gas containing a large amount of butadiene from which the solvent has been separated is produced. When condensing part of the stripped gas in the condenser, the condensed part is refluxed by returning it to the top of the stripping tower 8. Part of the uncondensed part is returned through a compressor 10 to the first extractive distillation tower 4, while the remainder is sent to a second extractive distillation tower 12. When condensing all of the stripped gas at the condenser, part of the condensed liquid is refluxed by returning it to the top of the stripping tower 8, part of the remainder is returned by the compressor 10 to the first extractive distillation tower 4, and the rest is sent to the second extractive distillation tower 12 by the compressor 10. What is returned to the first extractive distillation tower 4 is sometimes a vapor and sometimes liquid, but in both cases, the return ratio is controlled as explained later.

In the second extractive distillation tower 12, impurities having a volatility of butadiene or less (solubility of butadiene or more) are separated at the bottom of the tower. At the top of the tower, gas containing a high concentration of butadiene is taken out by holding the bottom pressure at an absolute pressure of 3 to 6 atm and holding the bottom temperature at 100 to 150°C. An extract containing a large amount of impurities separated at the bottom of the second extractive distillation tower 12 is led to the first stripping tower 13. At the first stripping tower 13, the bottom pressure is held at an absolute pressure of 1 to 3 atm and the bottom temperature is held at 120 to 180°C. The butadiene is separated from the extract and the stripped gas containing the butadiene is returned to the inlet of the condenser (not shown) of the stripping tower 8. The liquid at the bottom of the first stripping tower 13 is sent to the second stripping tower 14. At the second stripping tower 14, the bottom pressure is held at an absolute pressure of 1 to 3 atm and the bottom temperature is held at 150 to 200°C. The solvent is separated from the extract, exhausted from the bottom of the tower, and reused. The stripped gas is exhausted from the top of the tower.

The distillation gas containing a large amount of butadiene taken out from the top of the second extractive distillation tower 12 is successively sent to a topping tower 16 and a tailing tower 18. At the topping tower 16, the methylacetylene as impurity having a lower boiling point than butadiene is removed by making the bottom pressure 3 to 7 atm and making the bottom temperature 30 to 60°C. Further, at the tailing tower 18, the impurities having a higher boiling point than butadiene, for example, cis-2-butene, 1,2-butadiene, and ethylacetylene, are removed by making the bottom pressure 3 to 7 atm and the bottom temperature 40 to 70°C. In the present embodiment, the concentration of the finally obtained butadiene (BD) becomes at least 99 percent.

Next, an explanation will be given of the control apparatus and control method of the first extractive distillation tower 4 according to the present embodiment based on FIGS. 2 to 5.

As shown in FIG. 2, a feedstock feed line 20 to which a feedstock BBF containing butadiene is fed is connected to an intermediate stage of the first extractive distillation tower 4. The feedstock feed line 20 has a feedstock flow rate control valve (feedstock feed rate control means) 21a for controlling the flow rate of the feedstock fed to the first extractive distillation tower 4 attached to it. The feedstock flow rate control valve 21a is controlled in opening degree in accordance with the output signal from the load control means 62 and controls the flow rate of the feedstock fed through the feedstock feed line 20 to the first extractive distillation tower 4. While explained later, the present invention is characterized in the point of adjusting the opening degree of the feedstock flow rate control valve 21a and adjusting the feedstock BBF feed rate in accordance with the load conditions of each equipment detected through the load detecting means 61 and load control means 62.

Further, the feedstock feed line 20 has a feedstock flowmeter (feedstock feed rate detecting means) 21 attached to it to measure the flow rate of the feedstock fed to the first extractive distillation tower 4. The feedstock flow rate data measured by the feedstock flowmeter 21 is input to a load detecting means 61.

In the first extractive distillation tower 4, a solvent feed line 22 is connected to the top side of the feedstock feed line 20 and feeds the solvent for extraction of the butadiene to the inside of the first extractive distillation tower 4. The solvent feed line 22 has a solvent flowmeter (solvent ratio detecting means) 23 attached to it for measuring the flow rate of the solvent for extraction fed to the first extractive distillation tower 4. The solvent flow rate data measured by the solvent flowmeter 23 is input to the load detecting means 61. Further, the solvent feed line 22 has a solvent ratio control valve (solvent ratio control means) 23a attached to it, this controls the flow rate to the inside of the first extractive distillation tower 4 based on the output signal from the concentration predictive control means 60.

A reflux line 26 is connected to the top of the first extractive distillation tower 4 and takes out the residual gas remaining after extraction of the butadiene from the feedstock in the first extractive distillation tower 4 (however, containing some butadiene). This residual gas is condensed by the condenser 26a, and then part of the condensed residual gas is refluxed to the top of the inside of the first extractive distillation tower 4. The reflux line 26 has a reflux ratio meter (reflux ratio detecting means) 28 for measuring the flow rate of the residual gas after refluxing the residual gas taken out from the top of the first extractive distillation tower 4 again to the first extractive distillation tower 4. The reflux ratio data measured by the reflux ratio meter 28 is input to the load detecting means 61. Further, the reflux line 26 has a reflux ratio control valve (reflux ratio control means) 28a attached to it. The output signal from the concentration predictive control means 60 is used to control the opening degree and control the reflux ratio.

The reflux line 26 is also connected to the residual gas exhaust line 24. Part of the residual gas taken out at the reflux line 26 is exhausted to a not shown residual component tank. The residual gas exhaust line 24 has a residual gas distillation meter 29 attached to it for measuring the ratio of distillation of the residual gas BBR exhausted to the residual component tank. The distillation rate data measured by the residual gas distillation meter 29 is input to the load detecting means 61.

The reflux line 26 near the top of the first extractive distillation tower 4 has attached to it a target material concentration sensor (target material concentration detecting means) 25 for detecting the concentration of butadiene at the top of the tower and a top pressure sensor (top pressure detecting means) 27 for measuring the pressure inside the top of the tower. As the target material concentration sensor 25, for example, a gas chromatograph may be used. As the top pressure sensor 27, a general use pressure sensor may be used. The concentration data measured by the target material concentration sensor 25 is input to the concentration predictive control means 60. The pressure data measured by the top pressure sensor 27 is input to the load detecting means 61.

In the example shown in FIG. 2, a differential pressure sensor (differential pressure detecting means) 30 for detecting the pressure difference between the inside of the top of the tower and the inside of the bottom of the tower is attached to the first extractive distillation tower 4. In this example, the top-bottom differential pressure data detected by the differential pressure sensor 30 is input to the concentration predictive control means 60 and load detecting means 61.

First and second impurity concentration sensors (impurity concentration detecting means) 32 and 34 for measuring the concentration of the cis-2-butene and trans-2-butene and other impurities present in the seventh stage are attached to the seventh stage from the bottom of the first extractive distillation tower 4. The first impurity concentration sensor 32 detects the concentration of the cis-2-butene, while the second impurity concentration sensor 34 measures the concentration of the trans-2-butene. These concentration sensors 32 and 34 are not particularly limited so long as they can detect the concentrations of them, but for example are comprised of gas chromatographs. The data of the concentrations detected by these concentration sensors 32 and 34 are input to the concentration predictive control means 60.

Note that the positions of the first sensor and second sensor are not limited to the seventh stage from the bottom of the first extractive distillation tower 4. For example, they may be around the 15th stage from the bottom, on the third extraction line 44 from the stripping tower 8, or at the condensate line 51 as well. While the concentration data will not match at these locations, there is a strong correlation in the amounts of change of the concentrations. If continuously measuring the concentrations at any of these locations, it is possible to accurately judge the concentrations at the other locations. Further, since the concentration predictive control means 60 uses the concentration data converted to data of the change of concentration, if the change in concentration at these locations can be accurately measured, accurate concentration predictive control is possible.

At the bottom of the first extractive distillation tower 4, a reboiler (bottom temperature control means) 36a for controlling the bottom temperature is provided. The heat source of the reboiler 36a is not limited to steam. Hot water, a heating medium, etc. may also be illustrated, but in the present embodiment, the case of utilizing steam is illustrated. The reboiler 36a has a steam line 36b connected to it. This line 36b is equipped with a steam flowmeter (steam rate detecting means) 57 for measuring the flow rate of steam fed to the reboiler 36a, a steam rate control valve (steam rate control means) 57a, and a steam pressure meter (steam pressure detecting means) 58 for measuring the steam pressure. The flow rate data measured by the steam flowmeter 57 and the pressure data measured by the steam pressure meter 58 are input to the load detecting means 61. The steam rate control valve 57a controls in opening degree by the output signal corresponding to the concentration change data from the concentration predictive control means 60 so as to control the bottom temperature. The bottom temperature, as explained above, is generally held at 100 to 160°C, but in the present embodiment, the bottom temperature is controlled in this temperature range based on the output signal from the concentration predictive control means 60.

At the bottom of the first extractive distillation tower 4, a first extraction line 38 is connected. The extract (containing a solvent) containing a high concentration of butadiene present at the bottom of the tower is sent to the stripping tower 8. The stripping tower 8, as explained above, separates the solvent from the extract and exhausts it from the bottom. At the top of the stripping tower 8, stripped gas containing a large amount of butadiene from which the solvent has been separated is produced. This gas passes from the top through the third extraction line 44 by a compressor 10 to the second extractive distillation tower 12.

The third extraction line 44 has a stripped gas flowmeter (stripped gas flow rate detecting means) 54 for measuring the flow rate of the stripped gas or condensate of the stripped gas sent to the second extractive distillation tower 12 attached to it. The flow rate data measured by the stripped gas flowmeter 54 is input to the load detecting means 61.

A return line 46 is connected to the third extraction line 44. The return line 46 is connected to a stage near the bottom of the first extractive distillation tower 4. The stripped gas containing a large amount of butadiene carried through the third extraction line 44 or its condensate is returned to the inside of the first extractive distillation tower 4 through the return line 46.

The return line 46 is provided with a return flowmeter (return ratio detecting means) 50 for measuring the flow rate in the line and a return ratio control valve (return ratio control means) 48 for controlling the flow rate of the fluid flowing through the line. The return ratio data measured by the return flowmeter 50 is input to the load detecting means 61. The return ratio control valve 48 is controlled in accordance with an output signal from the concentration predictive control means 60 and controls the flow rate of the fluid returned to the inside the first extractive distillation tower 4 through the return line 46. When returning part of the stripped gas to the first extractive distillation tower 4 as a gas, the remainder of the stripped gas is sent to the second extractive distillation tower 12 while controlling the value of the pressure sensor 52 by the control valve 56. When returning the condensate of the stripped gas to the first extractive distillation tower 4, the remainder of the condensate is sent to the second extractive distillation tower 12 while controlling the liquid level of a condensate drum by the control valve 56.

### Load Control Method

In the present embodiment, the method of control using the load control means 62 shown in FIG. 2 will be explained based on FIG. 3.

When the control starts at step S1 shown in FIG. 3, at step S2, the load detecting means 61 shown in FIG. 2 reads the data CVi (i=5 to 14). CV5 is the data of the reflux ratio measured by the reflux ratio meter 28, CV6 is the data of the distillation rate measured by the residual gas distillation meter 29, CV7 is the data of the flow rate measured by the steam flowmeter 57, CV8 is the data of the pressure measured by the steam pressure meter 58, CV9 is the data of the return ratio measured by the return flowmeter 50, CV10 is the data of the flow rate measured by the stripped gas flowmeter 54, CV11 is the data of the solvent flow rate measured by the solvent flowmeter 23, CV12 is the data of the feedstock flow rate measured by the feedstock flowmeter 21, CV13 is the data of the pressure measured by the top pressure sensor 27, and CV14 is the data of the top-bottom differential pressure detected by the differential pressure sensor 30.

The load detecting means 61 stores a program able to calculate the load measurement values DAi (i=1 to 5) of each equipment based on the data CV5 to CV14.

Next, at step S3, the load measurement values DA1 to DA5 of each equipment are calculated based on the data CV5 to CV14. Specifically, the load measurement value DA1 of the condenser 26a and the load measurement value DA2 of the pump 26b are calculated from CV5 and CV6, the load measurement value DA3 of the reboiler 36a is calculated from CV7 or CV8, the load measurement value DA4 of compressor 10 is calculated from CV9 and CV10, and the load measurement value DA5 of the first extractive distillation tower 4 is calculated from CV5, CV6, CV7, CV9, CV11, CV12, CV13, and CV14.

Next, at step S4, the load control means 62 shown in FIG. 2 reads the load measurement values DA1 to DA5 of each equipment calculated by the load detecting means 61. The load control means 62 is comprised of a specific electric circuit having a memory circuit, a general use personal computer, a general use computer, a large-sized computer, etc. and stores a program for the later explained control. Note that instead of this program, use may be made of a logic circuit performing this operation.

The load control means 62 receives as input the load upper limit values DCi (i=1 to 5) for each equipment and the deviation allowable value Ai of the DCi and the above DAi. Specifically, DC1 is input as the load upper limit value of the condenser 26a, DC2 as the load upper limit value of the pump 26b, DC3 as the load upper limit value of the reboiler 36a, DC4 as the load upper limit value of the compressor 10, and DC5 as the load upper limit value of first extractive distillation tower 4. A1 is input as the deviation allowable value of the condenser 26a, A2 as the deviation allowable value of the pump 26b, A3 as the deviation allowable value of the reboiler 36a, A4 as the deviation allowable value of the compressor 10, and A5 as the deviation allowable value of the first extractive distillation tower 4.

Next, at step S5, the differences (DCi-DAi) between the load upper limit values DCi preset for the data DA1 to DA5 and the load measurement values DAi are calculated.

Next, at step S6, it is confirmed if these differences (DCi-DAi) are 0 or more for all of the condenser 26a, pump 26b, reboiler 36a, compressor 10, and first extractive distillation tower 4 and if any one or more of them is within a predetermined range of the deviation allowable value Ai or less. Further, when the difference (DCi-DAi) is in the above predetermined range, it means that the rate of production is maximized, so the current state is maintained and the steps from step S2 are repeated.

As opposed to this, at step S6, when the value of (DCi-DAi) is larger than Ai for all of the condenser 26a, pump 26b, reboiler 36a, compressor 10, and first extractive distillation tower 4, the routine proceeds to step S7 where the value of (DCi-DAi) is made to become 0 to Ai by sending a signal to increase the flow rate (control parameter) of the feedstock BBF fed to the first extractive distillation tower 4 to the feedstock flow rate control valve 21a and increasing the ratio of the feedstock BBF. Conversely, when the value of (DCi-DAi) is smaller than 0 for any one or more of the condenser 26a, pump 26b, reboiler 36a, compressor 10, and first extractive distillation tower 4 as well, the routine proceeds to step S7 where the value of (DCi-DAi) is made to become 0 to A by sending a signal to reduce the flow rate (control parameter) of the feedstock BBF fed to the first extractive distillation tower 4 to the feedstock flow rate control valve 21a and reducing the ratio of the feedstock BBF.

By working the control method of a first extractive distillation tower 4 according to the present embodiment, it is possible to increase to the maximum extent the load of equipment which fluctuates along with time and possible to operate the equipment at all times in the state of the maximum load of equipment. On the other hand, with just maximizing the load, sometimes the concentration of the butadiene included in the extract taken out from the bottom of the tower will not be stabilized and as a result, the purity of the conjugated dienes cannot be raised in the subsequent processes. Therefore, in the present embodiment, in addition to the above load control, by the later explained concentration predictive control, the gas chromatography composition (composition according to analysis by gas chromatography) deviating due to the fluctuation in the feed rate of the feedstock BBF can be adjusted by advanced control such as shown in FIG. 5.

### Concentration Predictive Control Method

In the present embodiment, as one example, a method of control using the concentration predictive control means 60 shown in FIG. 2 will be explained based on FIGS. 4 and 5.

When the control starts at step S1 shown in FIG. 4, at step S2, the concentration predictive control means 60 shown in FIG. 2 reads the data CVi (i=1 to 4).

CV1 is the data of the concentration of the cis-2-butene detected by the impurity concentration sensor 32 at the seventh stage of the extractive distillation tower 4 shown in FIG. 2, CV2 is the data of the concentration of the trans-2-butene detected by the impurity concentration sensor 34 at the seventh stage of the extractive distillation tower 4 shown in FIG. 2, CV3 is the data of the concentration of the butadiene detected by the target material concentration sensor 25 attached to the top of the extractive distillation tower 4, CV4 (=CV14) is the top-bottom differential pressure data detected by the differential pressure sensor 30 of the extractive distillation tower 4.

Next, at step S3, the data CV1 to CV4 after t seconds are forecast from a control model stored in the concentration predictive control means 60 shown in FIG. 2 based on the data CV1 to CV4 and those values made FCV1 to FCV4. Note that "after t seconds" is not particularly limited, but is for example after 600 to 3600 seconds.

Next, at step S4, the difference Ai-(i=1 to 4) between the target value PCVi preset for every data CV1 to CV4 and the forecasted value FCVi is calculated.

Next, at step S5, it is confirmed if the difference Ai is in a predetermined range from -αi (minus allowable value) to +αi (plus allowable value). If the difference Ai is in the predetermined range, it means that the forecasted value FCVi of the CVi after t seconds is in the allowable range. Note that the allowable value αi is determined for each CVi, while not particularly limited, it is about 1 to 10 percent of the target value PCVi.

If all of the differences Ai are allowable values at step S5, the forecasted values FCVi of the CVi after t seconds are in the allowable range, so the control parameters MV1 to MV4 are maintained in their current states and the steps after step S2 are repeated.

If even one of the differences Ai is out of the allowable range at step S5, it means that the corresponding forecasted value FCVi is out of the allowable range, so the routine proceeds to step S6, where the current settings of the control parameters MVi are changed so as to change the forecasted value FCVi deviating from the allowable range in a direction entering the allowable range. For example, when desiring to control a forecasted value FCVi deviating from the allowable range in a direction lowering the value, the current settings of the control parameters MVi are changed in the directions of the arrows shown in FIG. 5. In FIG. 5, the upward facing arrows mean raising the current settings of the control parameters MVi.

For example, when the forecasted concentration value FCV1 of cis-2-butene at the seventh stage corresponding to the data CV1 detected by the concentration sensor 32 shown in FIG. 2 rises out of the predetermined range, the forecasted concentration value FCV1 of cis-2-butene at the seventh stage is lowered by changing the current settings of the control parameters MVi as follows: That is, the concentration predictive control means 60 shown in FIG. 2 is used to operate the control valve 48 to increase the return ratio MV1 to the first extractive distillation tower 4 through the return line 46. Further, the concentration predictive control means 60 shown in FIG. 2 is used to control the control valve 23a to reduce the ratio of the solvent MV2 fed to the first extractive distillation tower 4 through the solvent feed line 22. Further, the concentration predictive control means 60 shown in FIG. 2 is used to control the control valve 28a of the reflux line 26 to reduce the reflux ratio MV3. Further, the concentration predictive control means 60 shown in FIG. 2 is used to control the reboiler 36a to increase the bottom temperature MV4.

Similarly, when the forecasted value of the concentration FCV2 of trans-2-butene at the seventh-stage corresponding to the data CV2 detected by the concentration sensor 34 shown in FIG. 2 rises out of the predetermined range, the FCV2 is lowered by changing the current settings of the control parameters MVi in accordance with the directions of the arrows shown in FIG. 5. Similarly, when the forecasted value of the concentration FCV3 of the butadiene at the top of the tower corresponding to the data CV3 detected by the target material concentration sensor 25 shown in FIG. 2 rises out of the predetermined range, the FCV3 is lowered by changing the current settings of the control parameters MVi in accordance with the directions of the arrows shown in FIG. 5. When the forecasted value FCV4 of the top-bottom differential pressure corresponding to the data CV4 detected by the differential pressure sensor 30 shown in FIG. 2 rises out of the predetermined range, it is preferable in terms of safety to lower this FCV4 by changing the current settings of the control parameters MVi in accordance with the directions of the arrows shown in FIG. 5. Note that when the forecasted values FCV1 to FCV4 corresponding to the data CV1 to CV4 drop out of the predetermined ranges, the control parameters MVi are controlled by the predictive control means 60 in directions opposite to the arrows shown in FIG. 5.

By this concentration predictive control, it is possible to reduce the variation in the concentration CV1 of the cis-2-butene at the seventh stage to about 0.63 percent (min. 8.50 - max. 9.13%), the variation in the concentration CV2 of the trans-2-butene at the seventh stage to about 0.32 percent (min. 1.35 - max. 1.67%), and the variation in the concentration CV3 of the butadiene at the top of the tower to about 0.21 percent (min. 0.19 - max. 0.40%).

### Action of Present Embodiment

As explained above, by working the control method of the first extractive distillation tower 4 according to the present embodiment, it is possible to increase to the maximum extent the load of the facilities which fluctuate along with time and possible to operate the equipment at all times in the state of the maximum load. Due to this, there is the merit that the efficiency of separation and purification is remarkably improved. Further, in the present embodiment, as explained above, predictive control of the concentration is performed, so by suppressing the fluctuations in the concentrations CV1 and CV2 of the cis-2-butene and trans-2-butene as impurities near the bottom of the first extractive distillation tower 4 and the fluctuations in the concentrations of butadiene at the top of the tower, it is possible to stabilize the concentration of butadiene contained in the extract taken out from the bottom of the tower. As a result, in the subsequent processes, increasing the purity of the conjugated dienes is easy and high purity conjugated dienes can be stably obtained. That is, in the present embodiment, it is possible to obtain high purity conjugated dienes stably and efficiently.

### Other Embodiments

Above, an embodiment of the present invention was explained, but the present invention is not limited to this embodiment in any way and may of course by worked in various ways within the range not exceeding the gist of the present invention.

In the above embodiment, the explanation was given illustrating the first extractive distillation tower 4, but it is most preferable to control the (DCi-DAi) values of all equipment of the second extractive distillation tower, topping tower, and tailing tower comprised of a condenser, pump, reboiler, compressor, and distillation tower to 0 or more and control one or more of them to Ai or less.

In the above embodiment, the load measurement values and load upper limit values for all equipment such as the condensers, pumps, reboilers, compressors, and distillation towers of the first extractive distillation tower, second extractive distillation tower, topping tower, and tailing tower were compared, but when it is clear that the value of (DCi-DAi) is sufficiently large and is over 0, the input to the load detecting means 61 and calculation can be omitted.

Further, in the above embodiment, the explanation was given of the process of separation and purification of unsaturated hydrocarbons of conjugated dienes, but the invention may also be applied to the case of separation and purification of unsaturated hydrocarbons other than conjugated dienes. For example, there is the case of purification and separation of butenes using as a feedstock the residual gas BBR produced as a byproduct in the process of separation and purification of conjugated dienes from the C₄ fraction or C₅ fraction of the above example. The control apparatus and control method of the extractive distillation tower in this case is similar to the case of the first extractive distillation tower 4 shown in FIG. 2 except that the feedstock fed is changed from the feedstock BBF to a residual gas BBR, the gas exhausted from the top is changed from the residual gas BBR to a gas containing butanes, the impurities detected as CV1 and CV2 are changed from cis-2-butene and trans-2-butene to n-butane and other butanes, and the unsaturated hydrocarbon in the extract extracted from the bottom of the extractive distillation tower is changed from conjugated dienes to butenes and can exhibit actions and effects similar to the above example.

## Claims

1. A separation and purification apparatus for an unsaturated hydrocarbon comprising:
an extractive distillation tower fed with feedstock containing unsaturated hydrocarbons and a solvent and distilling the feedstock and solvent to separate and purify a target unsaturated hydrocarbon which is part of the unsaturated hydrocarbons;
an impurity concentration detecting means for detecting a concentration of a specific impurity other than the target unsaturated hydrocarbon at the extractive distillation tower or another tower connected to the extractive distillation tower;
a target material concentration detecting means for detecting a concentration of the target unsaturated hydrocarbon at the extractive distillation tower or another tower connected to the extractive distillation tower;
a means for taking out a fluid containing the target unsaturated hydrocarbon from the bottom of the extractive distillation tower, a means for returning part of the fluid taken out to the extractive distillation tower, and a return ratio control means for controlling a return ratio returned to the extractive distillation tower;
a solvent ratio control means for controlling a feed rate of the solvent;
a reflux ratio control means for taking out a residual component of the feedstock from a top of the extractive distillation tower and controlling a reflux ratio of the residual component refluxed to the extractive distillation tower;
a bottom temperature control means for controlling a bottom temperature of the extractive distillation tower;
a concentration predictive control means for calculating a forecasted value of the concentration of the specific impurity and a forecasted value of the concentration of the target unsaturated hydrocarbon after a predetermined time based on values detected by the impurity concentration detecting means and the target material concentration detecting means and controlling the return ratio control means and the reflux ratio control means based on the forecasted values;
a feedstock feed rate control means for controlling the rate of the feedstock fed to the extractive distillation tower;
a load detecting means for detecting a load of the extractive distillation tower; and
a load control means for controlling the feedstock feed rate control means in accordance with detection values detected by the load detecting means.

2. A separation and purification apparatus for an unsaturated hydrocarbon comprising:
an extractive distillation tower fed with feedstock containing unsaturated hydrocarbons and a solvent and distilling the feedstock and solvent to separate and purify a target unsaturated hydrocarbon which is part of the unsaturated hydrocarbons;
an impurity concentration detecting means for detecting a concentration of a specific impurity other than the target unsaturated hydrocarbon at the extractive distillation tower or another tower connected to the extractive distillation tower;
a target material concentration detecting means for detecting a concentration of the target unsaturated hydrocarbon at the extractive distillation tower or another tower connected to the extractive distillation tower;
a means for taking out a fluid containing the target unsaturated hydrocarbon from the bottom of the extractive distillation tower, a means for returning part of the fluid taken out to the extractive distillation tower, and a return ratio control means for controlling a return ratio returned to the extractive distillation tower;
a solvent ratio control means for controlling a feed rate of the solvent;
a reflux ratio control means for taking out a residual component of the feedstock from a top of the extractive distillation tower and controlling a reflux ratio of the residual component refluxed to the extractive distillation tower;
a bottom temperature control means for controlling a bottom temperature of the extractive distillation tower;
a concentration predictive control means for calculating a forecasted value of the concentration of the specific impurity and a forecasted value of the concentration of the target unsaturated hydrocarbon after a predetermined time based on values detected by the impurity concentration detecting means and the target material concentration detecting means and controlling the return ratio control means, the solvent ratio control means, the reflux ratio control means, and the bottom temperature control means based on the forecasted values;
a feedstock feed rate control means for controlling the rate of the feedstock fed to the extractive distillation tower;
a load detecting means for detecting a load of the extractive distillation tower; and
a load control means for controlling the feedstock feed rate control means in accordance with a detection value detected by the load detecting means.

3. A method for separation and purification of an unsaturated hydrocarbon comprising the steps of:
distilling a feedstock containing a target unsaturated hydrocarbon and a solvent fed to an extractive distillation tower;
detecting a concentration of a specific impurity other than the target unsaturated hydrocarbon at the extractive distillation tower or another tower connected to the extractive distillation tower;
detecting a concentration of the target unsaturated hydrocarbon at the extractive distillation tower or another tower connected to the extractive distillation tower;
controlling a return ratio of part of a fluid containing the target unsaturated hydrocarbon taken out from a bottom of the extractive distillation tower and returned to the extractive distillation tower;
controlling a solvent ratio of the solvent fed to the extractive distillation tower;
controlling a reflux ratio of part of a residual component of the feedstock taken out from a top of the extractive distillation tower and refluxed to the extractive distillation tower;
controlling a bottom temperature of the extractive distillation tower;
calculating a forecasted value of the concentration of the specific impurity and a forecasted value of the concentration of the target unsaturated hydrocarbon after a predetermined time based on values detected by the impurity concentration detecting step and the target material concentration detecting step and controlling the return ratio and the reflux ratio based on the forecasted values;
controlling the feedstock feed rate of the feedstock fed to the extractive distillation tower;
detecting a load of the extractive distillation tower; and
controlling the feedstock feed rate in accoradance with a detection value detected by the load detection step.

4. A method for separation and purification of an unsaturated hydrocarbon comprising the steps of:
distilling a feedstock containing a target unsaturated hydrocarbon and a solvent fed to an extractive distillation tower;
detecting a concentration of a specific impurity other than the target unsaturated hydrocarbon at the extractive distillation tower or another tower connected to the extractive distillation tower;
detecting a concentration of the target unsaturated hydrocarbon at the extractive distillation tower or another tower connected to the extractive distillation tower;
controlling a return ratio of part of a fluid containing the target unsaturated hydrocarbon taken out from a bottom of the extractive distillation tower and returned to the extractive distillation tower;
controlling a solvent ratio of the solvent fed to the extractive distillation tower;
controlling a reflux ratio of part of a residual component of the feedstock taken out from a top of the extractive distillation tower and refluxed to the extractive distillation tower;
controlling a bottom temperature of the extractive distillation tower;
calculating a forecasted value of the concentration of the specific impurity and a forecasted value of the concentration of the target unsaturated hydrocarbon after a predetermined time based on values detected by the impurity concentration detecting step and the target material concentration detecting step and controlling the return ratio, the solvent ratio, the reflux ratio, and the bottom temperature based on the forecasted values;
controlling the feedstock feed rate of the feedstock fed to the extractive distillation tower;
detecting a load of the extractive distillation tower; and
controlling the feedstock feed rate in accordance with a detection value detected by the load detection step.

5. The method for separation and purification as set forth in claim 3 or 4, wherein the solvent is an amide compound.

6. The method for separation and purification as set forth in claim 3 or 4, wherein the feedstock is a C₄ fraction or C₅ fraction.

7. The method for separation and purification as set forth in claim 3 or 4, wherein the target unsaturated hydrocarbon is a conjugated diene.

8. The method for separation and purification as set forth in claim 3 or 4, wherein the target unsaturated hydrocarbon is butadiene or isoprene.
